(19) Europäisches Patentamt

European Patent Office

Office européen des brevets

(11) **EP 1 610 116 A2**

(12) **EUROPEAN PATENT APPLICATION**

(43) Date of publication:
**28.12.2005 Bulletin 2005/52**

(51) Int Cl.7: **G01N 21/45**

(21) Application number: **05012580.6**

(22) Date of filing: **10.06.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **16.06.2004 KR 2004044505**

(71) Applicant: SAMSUNG ELECTRONICS CO., LTD.
Suwon-si, Gyeonggi-do (KR)

(72) Inventors:
• **Shim, Jeo-young**
**Yongin-si, Gyeonggi-do (KR)**
• **Namgoong, Ji-na**
**Yongin-si, Gyeonggi-do (KR)**
• **Hwang, Kyu-youn**
**Incheon-si (KR)**

(74) Representative: **Grünecker, Kinkeldey,**
**Stockmair & Schwanhäusser Anwaltssozietät**
**Maximilianstrasse 58**
**80538 München (DE)**

(54) **Microstructured substrate having patterned thin layer, array of microstructures comprising the thin layer and methods of producing the microstructured substrate and the array of microstructures**

(57) A microarray substrate having a patterned thin layer on a substrate is provided. In the microarray substrate, the substrate has a reflectance different from that of a material of the thin layer, and the patterned thin layer includes a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

## FIG. 1

**Description**

BACKGROUND OF THE INVENTION

1. Field of the Invention

The present invention relates to a microarray substrate having a high ratio of spot signal to background signal in the course of optical detection, a microarray comprising the same, and methods of producing the microarray substrate and the microarray.

2. Description of the Related Art

A substrate conventionally used in the optical analysis method of a microarray was not surface-treated, or often used gratings in order to increase signal-to-noise ratio (hereinafter, also referred to as "SNR") to be obtained. For example, U.S. Patent No. 6,483,096 provided a method of increasing the SNR by separating excitation light and emission light using a circular grating structure.

However, in this conventional method, a lot of expenses were consumed to form gratings and the emission light was measured as guided light with a relatively low intensity. Thus, the need to improve the SNR still remained.

While intensively studying to resolve the above problems, the inventors unexpectedly discovered that an oxide layer with a specific thickness increases the intensity of the optical signal and an oxide layer with another specific thickness attenuates the intensity of the optical signal. Based on this fact, it was discovered that a spot region of a microarray is allowed to have a thin layer pattern in which constructive interference occurs and a background region is allowed to have a thin layer pattern in which destructive interference occurs, thereby improving the sensitivity of the optical signal produced from a microarray.

SUMMARY OF THE INVENTION

The present invention provides a microarray substrate used to produce a microarray that can increase the ratio of spot signal to background signal in the course of optical detection.

The present invention also provides a microarray that can increase the ratio of spot signal to background signal in the course of optical detection.

The present invention also provides a method of producing a microarray substrate used to produce a microarray that can increase the ratio of spot signal to background signal in the course of optical detection.

The present invention also provides a method of producing a microarray that can increase the ratio of spot signal to background signal in the course of optical detection.

According to an aspect of the present invention, there is provided a microarray substrate having a patterned thin layer on a substrate, wherein the substrate has a reflectance different from that of a material of the thin layer, and the patterned thin layer includes a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

According to another aspect of the present invention, there is provided a microarray comprising the microarray substrate of the present invention.

According to another aspect of the present invention, there is provided a method of producing the microarray substrate of the present invention, the method comprising: coating on a substrate a thin layer material having a reflectance different from that of the substrate to form a thin layer; and

patterning the thin layer to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

According to another aspect of the present invention, there is provided a method of producing the microarray of claim 8, the method comprising:

coating on a substrate a thin layer material having a reflectance different from that of the substrate to form a thin layer;
patterning the thin layer to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer; and

immobilizing biomolecules on the spot region.

BRIEF DESCRIPTION OF THE DRAWINGS

The above and other features and advantages of the present invention will become more apparent by describing in detail exemplary embodiments thereof with reference to the attached drawings in which:

FIG. 1 is a schematic diagram of a microarray substrate or a microarray of the present invention;
FIG. 2 is a plan view of the microarray substrate or the microarray of FIG. 1;
FIG. 3 illustrates the effect of an oxide layer pattern on a fluorescence intensity when the thickness of an oxide layer is 500 Å ;
FIG. 4 illustrates the effect of an oxide layer pattern on a fluorescence intensity when the thickness of an oxide layer is 1,000 Å ;
FIG. 5 illustrates the effect of an oxide layer pattern on a fluorescence intensity when the thickness of an oxide layer is 1,500 Å ;
FIG. 6 illustrates the effect of an oxide layer pattern on a fluorescence intensity when the thickness of an oxide layer is 2,000 Å ; and
FIG. 7 illustrates the effect of an oxide layer pattern on a fluorescence intensity of a microarray when the thickness of an oxide layer is 1,000 Å .

DETAILED DESCRIPTION OF THE INVENTION

The present invention provides a microarray substrate having a patterned thin layer on a substrate, in which the substrate has a reflectance different from that of a material of the thin layer, and the patterned thin layer includes a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

In the present invention, the substrate is any material, which may be used as a layer, including polymers, metals, ceramics, oxides, and the like. An array is formed on or supported by the surface of the substrate. The substrate may be an inorganic material, organic material, composite, or polymer. Examples of the substrate include, but are not limited to, glass, silicon wafer, fused silica, gold, silver, copper, platinum, polystyrene, poly(methylacrylate), and polycarbonate. The surface of the substrate may be modified, and adsorption, chemical reactions, or physical interactions may occur between the modified surface and an element of an array, which can support, promote, or catalyse the formation of the array.

In the present invention, the thin layer is composed of any material capable of transmitting light at a specific wavelength. Examples of a material for the thin layer include, but are not limited to, various metal oxides, such as silicon dioxide ($SiO_2$), glass, ceramic, mica, $Al_2O_3$, $TiO_2$, ITO, polystyrene, poly(methylacrylate), and polycarbonate.

The thin layer is patterned to include a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer. As used herein, the term "spot region" refers to a region in which biomolecules are immobilized, as used in common biomolecule microarrays. The term "background region" refers to a region in which biomolecules are not immobilized, or although they are immobilized, they are not detected in the course of detection after they bind to target molecules. The thickness of the spot region refers to a thickness at which constructive interference occurs between a first reflected light of irradiated excitation light reflected from the substrate and a second reflected light of irradiated excitation light reflected from the thin layer. This constructive interference amplifies light signals produced by the spot region. Thus, in the present invention, "the thickness occurring constructive interference" is not a specific numerical value but a certain range of a thickness in which the first reflected light and the second reflected light are reinforced to increase the intensity. Similarly, in the present invention, the term "the thickness occurring destructive interference" is not a specific numerical value but a certain range of a thickness in which the first reflected light and the second reflected light are cancelled. The surface of the thin layer may be modified and adsorption, chemical reactions, or physical interactions may occur between the modified surface and an element of an array, which can support, promote, or catalyse the formation of the array.

In the present invention, the reflectance of the substrate is greater than that of the thin layer and the constructive interference occurs under the condition that path difference, 2nsinθ d, of the first reflected light and the second reflected light is as follows:

[Equation 1 ]

$$2n \sin \theta d = (2m + 1)\lambda/2 (m = 0,1,2,3,...)$$

In equation 1, d is a thickness of the spot region, n is reflectance, θ is an incidence angle of an incident light, and λ is a wavelength. According to equation 1, when a path difference of the first reflected light and the second reflected light is an odd multiple of half a wavelength, the constructive interference occurs. If the wavelengthλ is 532 nm, the reflectance n is 1.462, and the incidence angle θ is 90°, the constructive interference occurs when the thickness of the spot region d is 91 nm, 273 nm, and the like.

The destructive interference occurs under the condition that a path difference, 2nsinθ d, of the first reflected light and the second reflected light is as follows:

[Equation 2]

$$2n \sin \theta d = 2m\lambda/2 (m = 0,1,2,3,...)$$

If the wavelength λ is 532 nm, the reflectance n is 1.462, and the incidence angle θ is 90°, the destructive interference occurs when the thickness of the space region d is 0 nm, 182 nm, and the like.

In an embodiment of the present invention, the substrate is silicon or glass and the patterned thin layer is silicon dioxide ($SiO_2$) (reflectance = 1.462). The thickness of the spot region is in the range of 500-1,500 Å and the thickness of the background region is in the range of 0-10 Å or 1,900-2,100 Å . A method of forming the $SiO_2$ layer on the silicon or glass layer is well known in the art. For example, the $SiO_2$ layer may be formed by depositing fused silica ($SiO_2$) on a silicon substrate by either dry or wet oxidation. In this case, the wavelength of excitation light may be 532 nm and a target material or a probe material may be labeled with Fluorescein, Cy3, or Cy5.

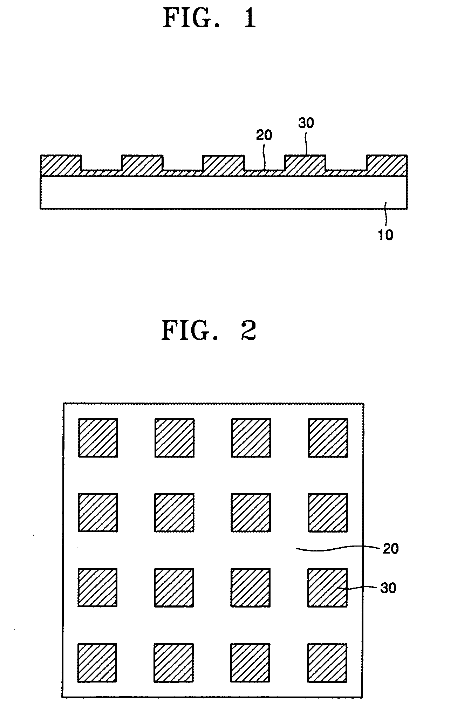

FIG. 1 schematically illustrates a microarray substrate or a microarray of the present invention. As illustrated in FIG. 1, the microarray substrate or the microarray 10 is patterned to have a spot region 30 and a background region 20. FIG. 2 is a plan view of the microarray substrate or the microarray of FIG. 1. Although the spot region is represented by a portion raised by patterning and the background region is represented by a lower portion in FIGS. 1 and 2, the background region may be represented by the portion raised by patterning and the spot region depending on reflectances of the thin layer and the substrate may be represented by the lower portion.

The present invention also provides a microarray including the microarray substrate on which biomolecules or other chemicals are immobilized. A process of immobilizing biomolecules on the microarray substrate is known in the art. Examples of the immobilization method include a method using photolithography and a method using spotting. According to the method using photolithography, a polynucleotide microarray may be prepared by repeating an operation of exposing to an energy source a certain region of the surface of a substrate which is coated with monomers protected by removable groups so as to remove the removable groups and an operation of coupling the monomers. In this case, a polynucleotide immobilized on the polynucleotide microarray is synthesized by extending monomers one at a time. Meanwhile, according to the spotting method, a microarray is prepared by immobilizing synthesized polynucleotides on fixed positions. These methods of preparing the polynucleotide are disclosed in, for example, U.S. Patent Nos. 5,744,305, 5,143,854, and 5,424,186. These literatures regarding a polynucleotide microarray and a method of preparing the same are incorporated herein in its entirety by reference.

In an embodiment of the microarray of the present invention, the biomolecule is selected from the group consisting of proteins, nucleic acids, and polysaccharides.

The present invention also provides a method of producing the microarray substrate, the method including: (a) coating on a substrate a thin layer material having a reflectance different from that of the substrate to form a thin layer; and (b) patterning the thin layer to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

In the operation (a) of the method of producing the microarray substrate of the present invention, a thin layer material having a reflectance different from that of the substrate is coated on the substrate to form a thin layer. The method of coating the thin layer is well known in the art. For example, a spin coating, gas phase or liquid phase depositing method, etc. may be used. A material and thickness used for the thin layer are the same as described above.

In the operation (b) of the method of producing the microarray substrate of the present invention, the obtained thin layer is patterned to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer. The thin layer may be patterned using any method well known in the art such as a photolithography. That is, a photoresist material is coated on the thin layer, the coated thin layer is then exposed to light through a patterned mask, and finally, shaped and etched to obtain the patterned thin layer having a spot region and a background region. In this case, the etching depth of the spot region is sufficient to cause constructive interference between the first reflected light and the second reflected light and the etching depth for the background region is sufficient to cause destructive interference between the first reflected light and the second reflected light. The condition causing the reinforcing or destructive interference is varied depending on the wavelength of irradiated excitation light and a difference in the reflectance between the substrate and the thin layer. The thickness condition causing the reinforcing or destructive interference may be calculated as equations 1 and 2 above.

The present invention also provides a method of producing the microarray, the method including: (a) coating on a substrate a thin layer material having a reflectance different from that of the substrate to form a thin layer; and (b) patterning the thin layer to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer; and (c) immobilizing biomolecules on the spot region.

In an embodiment of the method of the present invention, the biomolecule is selected from the group consisting of proteins, nucleic acids, and polysaccharides.

In the method of producing the microarray of the present invention, the operations of coating of the thin layer, patterning, and immobilizing biomolecules are the same as described above.

The present invention will now be described in greater detail with reference to the following examples. The following examples are for illustrative purposes and are not intended to limit the scope of the invention.

Examples

Example 1: Effect of the thickness of a thin layer on an optical signal

A $SiO_2$ layer with a thickness of 500-2,000 Å was formed on a silicon substrate and patterned using photolithography, and then a coupling agent was applied to the patterned $SiO_2$ layer. Then, the probe polynucleotides were immobilized on the substrate to obtain a microarray. Thereafter, to identify the effect of the thickness of the oxide layer on the detected signal, labelled target nucleic acids were hybridized with probe polynucleotides in the prepared microarray, excitation light was irradiated thereto, and then emission light produced therefrom was detected.

1. Formation of an oxide layer on a substrate

A substrate of silicon was used. An oxide layer with various thickness was formed on the substrate using a furnace SVF-200 apparatus (available from Seltron Inc.) by thermal oxidation. The thickness of the oxide layer was allowed to be 500-2,000 Å .

The thickness of the oxide layer was measured using NANOSPEC Model AFT 200 (available from NANOMETTICS Inc.). The NANOSPEC apparatus used the principle that when light was irradiated into a silicon wafer, some of the light is reflected by the oxide layer while some of the light passes through the oxide layer and is reflected by the silicon substrate. Here, the phase difference of the light reflected by the oxide layer and light reflected by the silicon substrate was used to determine the thickness of the oxide layer. The thickness of the oxide layer was measured by placing the silicon wafer on a sample stage of the NANOSPEC apparatus. The thickness was measured at 5-6 points in the wafer and an average value was derived therefrom to be used for coating. All experiments were performed in a clean room-class 1000 from which most dust particles are removed.

2. Formation of a thin layer pattern on the substrate

The $SiO_2$ oxide layer on the silicon wafer was patterned using photolithography. First, the surface of the silicon wafer was pretreated with hexamethyldisilazane (HMDS). This pretreatment was performed to increase the resolution of the pattern by increasing the adhesive force to a photoresist to be subsequently coated thereon. The HMDS was applied by a spin coating method using a spin coater model CEE 70 (available from CEE Inc.). The spin coating was performed by a initial coating at 500 rpm for 5 sec and a main coating at 4,000 rpm for 40 sec. Then, a positive photoresist, AZ1512 or AZ GXR 601, which was generally used in the semiconductor process, was spin coated on the HMDS using a spin coater model CEE 70 (available from CEE Inc.). The spin coating was performed by an initial coating at 500 rpm for 5 sec and a main coating at 4,000 rpm for 40 sec. Immediately after the spin coating was completed, the silicon wafer was soft-baked on a hot plate at 110°C for 2 minutes so as to increase the adhesive force of the photoresist to the surface of the silicon wafer.

Then, a photomask (made to order in PKL Inc.) on which a spot region and a background region were represented by a transparent region and an opaque region, respectively, was arranged on the coated photoresist , and then exposed to light through an EV 620 apparatus (available from Vision Inc.). The exposure was performed at the amount of light of 13 mJ for 4.5 seconds. After finishing the exposure, a development was performed using MIF 300K as a developing solution to form a photoresist pattern.

Thereafter, the photoresist pattern was etched using a fluoric acid solution or a buffered oxide etcher (BOE) (6:1 or 5:1) as an etching solution for 1-3 minutes. As a result, about 1,000-2,000 Å in the thickness of the silicon oxide layer ($SiO_2$) was selectively removed. Finally, a piranha solution of acetone/IPA or hydrogen peroxide and sulfuric acid (1:3) was used to remove the photoresist.

3. Coating of a fluorescent dye and identification of a constructive interference and destructive interference according to the thickness of the oxide layer

First, the substrate was thoroughly cleaned prior to the surface treatment. In the cleaning, a piranha solution of sulfuric acid and hydrogen peroxide (3: 1) was used to remove organic contaminants. Finally, the substrate was washed with a sufficient amount of water, and then dried. The cleaning of the substrate was performed using a wet station which was used in the semiconductor process, a sulfuric acid bath was used for the piranha solution, and the washing with water was performed using a QDR process. Each substrate was fixed to a silicon wafer carrier of a Teflon material throughout the process. The cleaned substrate was then spin-dried.

Immediately after the cleaning of the substrate was completed, a solution of 20% (vol/vol) GAPS (γ -aminopropyltriethoxy silane) in ethanol(GAPS: ethanol:5% FC-4430 surfactant in ethanol solution = 2:5:3(vol/vol)) was spin coated on the substrate having the silicon oxide layer pattern using a spin coater model CEE 70 (available from CEE Inc.). The spin coating was performed by an initial coating at 500 rpm for 10 sec and a main coating at 2,000 rpm for 10 sec. After the spin coating was completed, the substrate was fixed to a Teflon wafer carrier and incubated for 13 minutes, and then cured at 120°C for 40 minutes. The cured substrate was immersed in water for 10 minutes, ultrasonically washed, immersed again in water for 10 minutes, and then spin-dried. The dried substrate was cut to have a square or rectangular shape and used in the experiments. All experiments were performed in a clean room-class 1000 from which most dust particles were removed.

Fluorescein (0.05 g/10 ml) was dip-coated on the silanized substrate. First, Fluorescein was dissolved in a DMF solution to prepare an immersion solution (Fluorescein 0.05 g/10 ml). The immersion solution and the substrate were placed in a reaction container and reacted at 40 °C for 120 minutes. After completing the reaction, the substrate was removed from the immersion solution, and cleaned three times with DMF for 10 minutes, and then cleaned three times with methanol for 10 minutes. Thereafter, the substrate was dried, and then the amount of Fluorescein reacted with the substrate was determined using GenePix 4000B scanner (available from Axon Inc.).

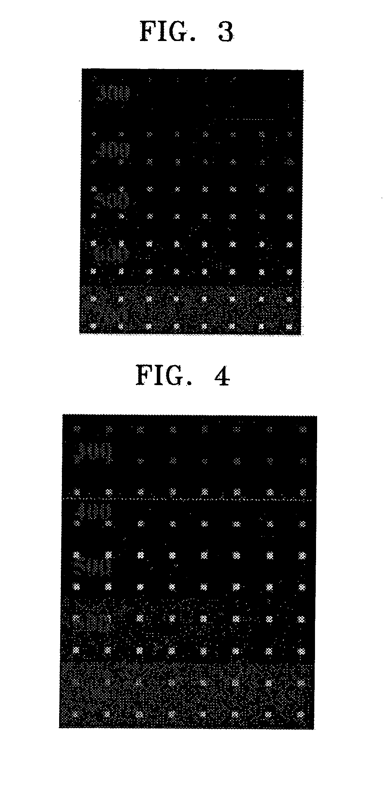

The obtained results are shown in FIGS. 3, 4, 5, and 6. FIG. 3 shows an effect of an oxide layer pattern on a fluorescence intensity when the thickness of the oxide layer is 500 Å . The result of FIG. 3 was obtained by patterning an oxide layer such that the thickness of a portion required for constructive interference (spot region) was 500 Å and the thickness of a portion required for destructive interference (background region) was 0-10 Å (the thickness of natural oxide layer); coating GAPS on the patterned oxide layer; coating Fluorescein thereon; and then irradiating light having a wavelength of 532 nm on the coated oxide layer and measuring a fluorescence intensity at a wavelength of 570 nm. The fluorescence intensity was measured while increasing the sensitivity by adjusting a photomultiplier tube (PMT) of a scanner within a range of 300-700. Numerical values in figures represent the PMT sensitivity. The fluorescence intensities of the spot region and the background region observed in FIG.3, and the ratio of the fluorescence intensity of the spot region to that of the background region, i.e., signal to noise ratio (SNR) calculated therefrom are shown in Table 1.

Table 1

| PMT sensitivity | FI of spot region | FI of background region | SNR |
|---|---|---|---|
| 300 | 49 | 35 | 1.4 |
| 400 | 215 | 37 | 6 |
| 500 | 1100 | 40 | 28 |
| 600 | 4300 | 51 | 84 |
| 700 | 12700 | 85 | 149 |
| FI: Fluorescence intensity | | | |

Referring to Table 1, as the PMT sensitivity increased from 300 to 700, the SNR increased from 1.4 to 149. In general microarrays, a SNR of 10 or more at a PMT sensitivity of 500 is regarded as a normal signal. Thus, the above results may be regarded as superior results.

FIG. 4 shows the effect of an oxide layer pattern on a fluorescence intensity when the thickness of the oxide layer is 1,000 Å. The result of FIG. 4 was obtained by patterning an oxide layer such that the thickness of a portion required for constructive interference (spot region) was 1,000 Å and the thickness of a portion required for destructive interference (background region) was 0-10 Å; coating GAPS on the patterned oxide layer; coating Fluorescein thereon; and then irradiating light having a wavelength of 532 nm on the coated oxide layer in order to measure a fluorescence intensity at a wavelength of 570 nm. The fluorescence intensities of the spot region and the background region observed in FIG. 4, and the ratio of the fluorescence intensity of the spot region to that of the background region, i.e., SNR calculated therefrom are shown in Table 2.

Table 2

| PMT sensitivity | FI of spot region | FI of background region | SNR |
|---|---|---|---|
| 300 | 140 | 36 | 4 |
| 400 | 900 | 38 | 24 |
| 500 | 6700 | 41 | 165 |
| 600 | 26500 | 58 | 457 |
| 700 | 64800 | 110 | 589 |
| FI: Fluorescence intensity | | | |

Referring to Table 2, as the PMT sensitivity increased from 300 to 700, the SNR increased from 4 to 589, indicating that the results are superior.

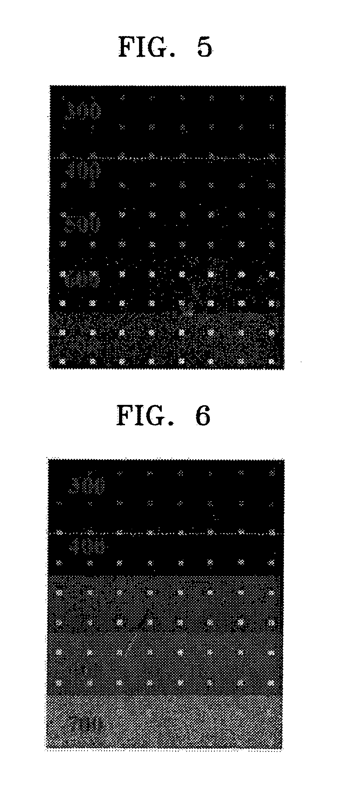

FIG. 5 shows the effect of an oxide layer pattern on a fluorescence intensity when the thickness of the oxide layer is 1,500 Å. The result of FIG. 5 was obtained by patterning an oxide layer such that the thickness of a portion required for constructive interference (spot region) was 1,500 Å and the thickness of a portion required for destructive interference (background region) was 0-10 Å; coating GAPS on the patterned oxide layer; coating Fluorescein thereon; and then irradiating light having a wavelength of 532 nm on the coated oxide layer and measuring a fluorescence intensity at a wavelength of 570 nm. The fluorescence intensities of the spot region and the background region observed in FIG. 5, and the ratio of the fluorescence intensity of the spot region to that of the background region, i.e., SNR calculated therefrom are shown in Table 3.

Table 3

| PMT intensity | FI of spot region | FI of background region | SNR |
|---|---|---|---|
| 300 | 60 | 36 | 1.7 |
| 400 | 300 | 38 | 8 |
| 500 | 2260 | 45 | 50 |
| 600 | 68700 | 60 | 115 |
| 700 | 20500 | 95 | 216 |

Table 3 (continued)

| PMT intensity | FI of spot region | FI of background region | SNR |
|---|---|---|---|
| FI: Fluorescence intensity | | | |

Referring to Table 3, as the PMT sensitivity increased from 300 to 700, the SNR increased from 1.7 to 216, indicating that the results are superior.

FIG. 6 shows an effect of an oxide layer pattern on a fluorescence intensity when the thickness of the oxide layer is 2,000 Å . The result of FIG. 6 was obtained by patterning an oxide layer such that the thickness of a portion required for constructive interference (spot region) was 1,000 Å and the thickness of a portion required for destructive interference (background region) was 2,000 Å ; coating GAPS on the patterned oxide layer; coating Fluorescein thereon; and then irradiating light having a wavelength of 532 nm to the coated oxide layer and measuring a fluorescence intensity at a wavelength of 570 nm. The fluorescence intensities of the spot region and the background region observed in FIG. 6, and the ratio of the fluorescence intensity of the spot region to that of the background region, i.e., SNR calculated therefrom are shown in Table 4.

Table 4

| PMT sensitivity | FI of spot region | FI of background region | SNR |
|---|---|---|---|
| 300 | 100 | 36 | 2.8 |
| 400 | 610 | 51 | 12 |
| 500 | 5600 | 130 | 43 |
| 600 | 21500 | 400 | 54 |
| 700 | 63500 | 1050 | 60 |
| FI: Fluorescence intensity | | | |

Referring to Table 4, as the PMT sensitivity increased from 300 to 700, the SNR increased from 2.8 to 60.

The increase in the SNR is most apparent in Table 2, indicating that to use the oxide layer of 1,000 Å has the highest efficient for constructive interference and the oxide layer of 0-10 Å for destructive interference.

Example 2: Preparation of a polynucleotide microarray and the effect of the thickness of an oxide layer on a fluorescence intensity

In this Example, probe polynucleotides were immobilized on a spot region of the patterned oxide layer having a thickness of 1,000 Å , prepared in Example 1, labeled target nucleic acids were hybridized, and then the fluorescence intensity was measured to investigate the effect of the thickness of an oxide layer on the fluorescence intensity of a microarray.

First, as described in Example 1, a patterned oxide layer having the thickness of a spot region of 1,000 Å and that of a background region of 0-10 Å was obtained, and probe polynucleotides were immobilized thereon. The immobilization of the probe polynucleotides was achieved by immobilizing polynucleotides on the substrate by a spotting process. The probe polynucleotides were added to a 100 mM $NaHCO_3$ (pH = 9.0) solution, and the resulting solution was left at 37°C for 1 hour. It was then used as a spotting solution. The spotting solution was spotted on the substrate, and then the spotted substrate was left in a wet chamber at 70°C under a relative humidity of 40% for 1 hour. Then, a process required for background control was performed. That is, in order to prevent the target nucleic acids from bonding to a glass surface, amine groups in an unspotted position of the substrate surface were allowed to be negatively charged, and then the substrate was stored in a drying machine. The target polynucleotide labeled with Cy-3 was hybridized with the probe polynucleotide in the prepared substrate, i.e., DNA chip, and then the fluorescence was measured at a wavelength of 532 nm.

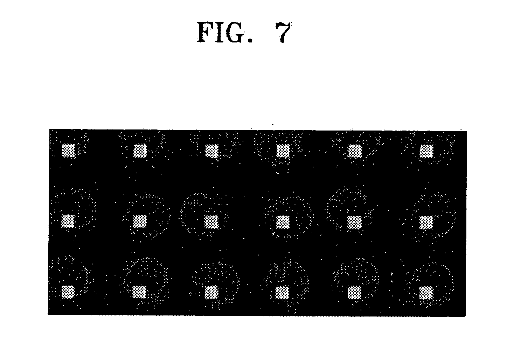

The result is shown in FIG. 7. FIG. 7 shows the effect of an oxide layer pattern on a fluorescence intensity when the thickness of the oxide layer is 1,000 Å . The result of FIG. 7 was obtained by patterning an oxide layer such that the thickness of a portion required for constructive interference (spot region) was 1,000 Å and the thickness of a portion required for destructive interference (background region) was 0-10 Å ; coating GAPS on the patterned oxide layer; spotting and immobilizing a probe polynucleotide (SEQ ID NO. 1) thereon; hybridizing the probe polynucleotide with a target polynucleotide (SEQ ID NO. 2) labeled with Cy-3; and then measuring a fluorescence at a wavelength of 532 nm. Referring to FIG. 7, the microarray having the patterned oxide layer with a thickness of 1,000 Å also has a fluorescence intensity signal of better SNR similar to the substrate having the patterned oxide layer with a thickness of 1,000 Å .

According to the microarray substrate of the present invention, a microarray having a high ratio of spot signal to background signal in the course of optical detection can be produced.

According to the microarray of the present invention, the ratio of spot signal to background signal in the course of optical detection is high.

According to the method of producing a microarray substrate of the present invention, a microarray substrate used to produce a microarray having a high ratio of spot signal to background signal in the course of optical detection can be produced.

According to the method of producing a microarray of the present invention, a microarray having a high ratio of spot signal to background signal in the course of optical detection can be produced.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A microarray substrate having a patterned thin layer on a substrate wherein the substrate has a reflectance different from that of a material of the thin layer, and the patterned thin layer includes a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

2. The microarray substrate of claim 1, wherein the substrate is selected from the group consisting of glass, silicon wafer, fused silica, gold, silver, copper, platinum, polystyrene, poly(methylacrylate), and polycarbonate.

3. The microarray substrate of claim 1, wherein the thin layer is selected from the group consisting of silicon dioxide ($SiO_2$), glass, lithium fluoride (LiF), silicon wafer, fused silica, ceramic, mica, $Al_2O_3$, $TiO_2$, ITO, polystyrene, poly (methylacrylate), and polycarbonate.

4. The microarray substrate of claim 1, wherein the reflectance of the substrate is greater than that of the material of the thin layer, the thickness of the spot region satisfies an equation of $d=(2m+1)\lambda/4n \sin\theta$ and the thickness of the background region satisfies an equation of $d=2m\lambda/4n \sin\theta$ , in which m is 0 or a positive integer, d is a thickness, n is a reflectance, $\theta$ is an incidence angle of an incident light, and $\lambda$ is a wavelength.

5. The microarray substrate of claim 1, wherein the substrate is silicon or glass and the patterned thin layer is $SiO_2$.

6. The microarray substrate of claim 5, wherein the thickness of the spot region is in a range of 500-1,500 Å .

7. The microarray substrate of claim 5, wherein the thickness of the background region is in a range of 0-10 Å or 1,900-2,000 Å .

8. A microarray comprising the microarray substrate of any one of claims 1 to 7.

9. The microarray of claim 8, wherein a biomolecule selected from the group consisting of proteins, nucleic acids, and polysaccharides is immobilized on the spot region.

10. A method of producing the microarray substrate of any one of claims 1 to 7, the method comprising:

    coating on a substrate a thin layer material having a reflectance different from that of the substrate to form a thin layer; and
    patterning the thin layer to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer.

11. A method of producing the microarray of claim 8, the method comprising:

    coating on a substrate a thin layer material having a reflectance different from that of the substrate to form a thin layer;

patterning the thin layer to form a spot region having a thickness at which constructive interference occurs between a first reflected light of irradiated excitation light reflected from the substrate and a second reflected light of irradiated excitation light reflected from the thin layer and a background region having a thickness at which destructive interference occurs between the first reflected light of irradiated excitation light reflected from the substrate and the second reflected light of irradiated excitation light reflected from the thin layer; and immobilizing biomolecules on the spot region.

12. The method of claim 11, wherein the biomolecule is selected from the group consisting of proteins, nucleic acids, and polysaccharides.

FIG. 1

FIG. 2

FIG. 3

FIG. 4

FIG. 5

FIG. 6

FIG. 7

Sequence Listing

<110>    Samsung Electronics Co. Ltd.

<120>    A substrate for microarray and a microarray having a patterned
         thin layer and a method for producing the substrate for
         microarray and the microarray

<130>    PN054286

<160>    2

<170>    KopatentIn 1.71

<210>    1
<211>    15
<212>    DNA
<213>    Artificial Sequence

<220>
<223>    probe polynucleotide coupled with NH2-(CH2)- group at it's 5'
         terminal

<400>    1
cggaggaacc gtttc                                                    15

<210>    2
<211>    15
<212>    DNA
<213>    Artificial Sequence

<220>
<223>    target polynucleotide labelled with Cy-3 at it's 3' terminal

<400>     2

gcctccttgg caaag                                                                                                   15